# EUROPÄISCHE PATENTSCHRIFT

(11) **EP 2 646 825 B1**
(45) Veröffentlichungstag und Bekanntmachung des Hinweises auf die Patenterteilung: **17.10.2018**
(21) Anmeldenummer: 11817311.1
(22) Anmeldetag: 30.11.2011
(51) Int. Cl.: G01N 33/558, G01N 33/569, G01N 33/68

(54) **SCHNELLTEST ZUM QUALITATIVEN UND/ODER QUANTITATIVEN BESTIMMEN VON IN KÖRPERFLÜSSIGKEIT ENTHALTENEN ANTIKÖRPERN GEGEN HUMANE PAPILLOMVIREN SOWIE VORRICHTUNG ZUM DURCHFÜHREN DES SCHNELLTESTS**
RAPID TEST FOR THE QUALITATIVE AND/OR QUANTITATIVE ANALYSIS OF ANTIBODIES AGAINST HUMAN PAPILLOMA VIRUSES (HPV) PRESENT IN BODY FLUID, AND DEVICE FOR CARRYING OUT THE RAPID TEST
TEST RAPIDE POUR LA DÉTERMINATION QUALITATIVE ET/OU QUANTITATIVE D'ANTICORPS CONTRE LES PAPILLOMAVIRUS HUMAINS CONTENUS DANS UN FLUIDE CORPOREL ET DISPOSITIF POUR LA MISE EN OEUVRE DU TEST RAPIDE

(30) Priorität: 03.12.2010 DE 102010061028
(43) Veröffentlichungstag der Anmeldung: 09.10.2013
(60) Teilanmeldung: 18185755.8
(73) Patentinhaber: Abviris Deutschland GmbH, 22949 Ammersbek (DE)
(72) Erfinder: HILFRICH, Ralf, 65597 Hünfelden (DE)
(74) Vertreter: Launhardt, Thomas
(86) Internationale Anmeldenummer: PCT/DE2011/075292
(87) Internationale Veröffentlichungsnummer: WO 2012/097788

(56) Entgegenhaltungen:
- EP-A1- 1 271 151
- WO-A1-97/02491
- WO-A1-98/23958
- US-A1- 2008 199 851
- US-B1- 6 358 744
- MyBioSource: "HPV16L1 antigen", , Dezember 2006 (2006-12), XP002672343, Gefunden im Internet: URL:http://www.mybiosource.com/datasheet.p hp?products_id=318636 [gefunden am 2012-03-26]
- OPALKA DAVID ET AL: "Simultaneous quantitation of antibodies to neutralizing epitopes on virus-like particles for human papillomavirus types 6, 11, 16, and 18 by a multiplexed luminex assay", CLINICAL AND DIAGNOSTIC LABORATORY IMMUNOLOGY, AMERICAN SOCIETY FOR MICROBIOLOGY, US, vol. 10, no. 1, 1 January 2003 (2003-01-01), pages 108-115, XP002421126, ISSN: 1071-412X, DOI: 10.1128/CDLI.10.1.108-115.2003
- D Skiba ET AL: "Prognostic Significance of Serum Antibodies to HPV-16 L1 Virus-like Particles in Patients with Invasive Cervical Cancer", Anticancer Research, 1 January 2006 (2006-01-01), pages 4921-4926, XP055155993, Retrieved from the Internet: URL:http://ar.iiarjournals.org/content/26/ 6C/4921.full.pdf [retrieved on 2014-12-01]

## Beschreibung

Die Erfindung betrifft einen Schnelltest zum qualitativen und/oder quantitativen Bestimmen von in Körperflüssigkeit enthaltenen Antikörpern gegen humane Papillomviren. Die Erfindung betrifft ferner eine Vorrichtung zur Durchführung eines solchen Schnelltests.

### Technologischer Hintergrund und Stand der Technik

Humane Papillomviren, abgekürzt HPV, bilden eine Gruppe von Viren, die mittlerweile in mehr als 100 Typen eingeteilt werden. Die Viren befallen die Epithelzellen der Haut oder verschiedener Schleimhäute und verursachen bei den infizierten Zellen ein unkontrolliertes, tumorartiges Wachstum. Die Tumore sind meist gutartig und führen lediglich zur Warzenbildung an der betroffenen Haut- oder Schleimhautstelle, an welcher die Infektion mit den Viren vorliegt.

Die humanen Papillomviren verursachen jedoch nicht nur gutartige Tumore. Einige HPV-Typen können auch bösartige Veränderungen hervorrufen. Beispielsweise stehen humane Papillomviren in Verdacht, bei der Entstehung von Gebärmutterhalskrebs, dem sogenannten Zervixkarzinom, beteiligt zu sein. Auch stehen die humanen Papillomviren in Verdacht, Scheiden-, Penis- und Analkarzinomen zu verursachen oder zumindest an deren Entstehung beteiligt zu sein.

Um einem solchen tumorartigen Wachstum vorzubeugen, wurden in den letzten Jahren Impfstoffe gegen humane Papillomviren entwickelt und in Deutschland zugelassen. Durch die Impfung wird die Wahrscheinlichkeit einer Infizierung der geimpften Person durch humane Papillomviren erheblich gesenkt. Die Impfung ist jedoch nur dann wirksam, sofern die Person danach auch Antikörper gegen humane Papillomviren aufweist und die Antikörper in entsprechender Konzentration vorhanden sind. Insofern kommt einem Nachweis über die Wirksamkeit der Impfung maßgebliche Bedeutung zu.

Bisher ist jedoch ein kommerziell verfügbares Nachweissystem für die HPV-Antikörper in Körperflüssigkeiten nicht vorhanden.

### Aufgabenstellung

Der Erfindung liegt daher die Aufgabe zugrunde, einen Schnelltest zum qualitativen und/oder quantitativen Bestimmen von in Körperflüssigkeit enthaltenen Antikörpern gegen humane Papillomviren mit den eingangs genannten Merkmalen bereitzustellen. Ferner soll eine Vorrichtung zur Durchführung eines solchen Schnelltests vorgeschlagen werden.

### Erfindung und vorteilhafte Wirkungen

Die Aufgabe wird mit einem Schnelltest zum qualitativen und/oder quantitativen Bestimmen von in Körperflüssigkeit enthaltenen Antikörpern gegen humane Papillomviren gelöst, welcher die Merkmale des Anspruches 1 aufweist. Die Aufgabe wird ferner mit einer Vorrichtung zur Durchführung eines Schnelltests zum qualitativen und/oder quantitativen Bestimmen von in Körperflüssigkeit enthaltenen Antikörpern gegen humane Papillomviren mit den Merkmalen des Anspruches 5 gelöst. Ferner wird zur Lösung der Aufgabe ein Behältnis mit den Merkmalen des Anspruches 10 und ein Analysesystem mit den Merkmalen des Anspruches 11 vorgeschlagen. Vorteilhafte Ausgestaltungen der Erfindung ergeben sich aus den Unteransprüchen, der nachfolgenden Beschreibung und den Figuren.

Ein erfindungsgemäßer Schnelltest zum qualitativen und/oder quantitativen Bestimmen von in Körperflüssigkeit enthaltenen Antikörpern gegen humane Papillomviren, im Zuge der Erfindung auch als HPV bezeichnet, sieht in einem ersten Verfahrensschritt vor, dass eine Probe von Körperflüssigkeit, insbesondere eine an einem Menschen entnommene Probe von Körperflüssigkeit, mit einem Reagenz gemischt wird, wobei das Reagenz im Wesentlichen aus einer vorgegebenen Menge an physiologisch wirkender Flüssigkeit, wie beispielsweise einer Kochsalzlösung, und einer vorgegebenen Menge wenigstens eines HPV-spezifischen Antigens besteht. In einem zweiten Verfahrensschritt wird dann das Gemisch aus dem Reagenz und der Probe von Körperflüssigkeit einer Analyse zugeführt, welche eine messbare und/oder von einem Benutzer wahrnehmbare Veränderung nutzt.

Durch den erfindungsgemäßen Schnelltest ist ein Nachweis von Antikörpern gegen HPV in einfacher Weise und mit wenig Aufwand erbringbar. Eine aufwendige und komplizierte Analyse in einem Zentrallabor ist nicht notwendig. Bei dem erfindungsgemäßen Schnelltest kann die diagnostische Untersuchung bereits vor Ort, also in der Praxis eines niedergelassenen Arztes oder im Krankenhaus unmittelbar auf der Krankenstation vorgenommen werden.

Um eine Analyse für den Nachweis von humanen Antikörpern gegen HPV durchführen zu können, ist nach der Erfindung der Verfahrensschritt vorgeschaltet, dass die an dem Menschen entnommene Probe an Körperflüssigkeit mit dem wenigstens einen HPV-spezifischen Antigen zusammengebracht wird, um bereits vor der Analyse einen Komplex aus dem Antigen und etwaigen in der Probe enthaltenen Antikörpern zu bilden. Sofern in der Probe die zu dem wenigstens einen Antigen zugehörigen Antikörper enthalten sind, wird das Reagenz inaktiviert. Sofern keine entsprechenden Antikörper in der Probe enthalten sind, bleibt das Reagenz reaktiv. Diese reaktive Eigenschaft des Reagenz wird dann bei der Analyse genutzt.

Es hat sich gezeigt, dass eine Verweilzeit der Probe von Körperflüssigkeit in dem Reagenz von etwa 10 Minuten ausreicht, um das Reagenz zu Inaktivieren oder nicht, je nachdem, ob Antikörper in der Probe enthalten sind oder nicht. Es kann dadurch der gesamte Schnelltest in kürzester Zeit durchgeführt werden, so dass beispielsweise belastbare Ergebnisse innerhalb einer Zeitdauer von weniger als einer Stunde nach der Probenahme vorliegen.

Maßgeblich ist es bei dem erfindungsgemäßen Schnelltest, dass das HPV-spezifische Antigen in dem Reagenz vorgehalten wird, ohne dass das HPV-spezifische Antigen seine Fähigkeit zum Reagieren mit Antikörpern gegen HPV einbüßt oder in seiner Reaktivität bezüglich der Antikörper nachlässt. Bei dem erfindungsgemäßen Schnelltest wird deshalb die physiologisch wirkende Flüssigkeit genutzt, welche aufgrund ihrer physiologischen Wirkungsweise weder kurzfristig noch langfristig einen negativen Einfluss auf das HPV-spezifische Antigen bzw. dessen Wirkungsweise im Hinblick auf Antikörper gegen HPV hat. Unter kurzfristig ist dabei ein Zeitraum bis etwa 4 bis 5 Wochen zu verstehen. Ferner ist unter langfristig ein Zeitraum bis etwa 2 bis 3 Jahren zu verstehen.

Als physiologisch wirkende Flüssigkeit kann beispielsweise TRIS-BSA mit einem pH-Wert von etwa 7,0 bis etwa 9,0, vorzugsweise 7,2 bis 7,4, zum Einsatz kommen. TRIS ist dabei die Abkürzung für Tris(hydroxymethyl)-aminomethan und BSA die Abkürzung für Bovines Serumalbumin. Eine solche physiologisch wirkende Flüssigkeit ist besonders stabil, so dass ein negativer Einfluss auf das HPV-spezifische Antigen dauerhaft vermieden ist.

Denkbar ist es auch, dass als physiologisch wirkende Flüssigkeit eine Kochsalzlösung, insbesondere mit einem pH-Wert von etwa 7,0 bis etwa 9,0, vorzugsweise 7,2 bis 7,4, zum Einsatz kommt.

Je nachdem, ob in der Probe von Körperflüssigkeit Antikörper gegen humane Papillomviren enthalten sind oder nicht, kommt es im Zuge der Analyse zu einer messbaren und/oder von einem Benutzer wahrnehmbaren Veränderung oder es bleibt eine solche Veränderung aus. Bei dem erfindungsgemäßen Schnelltest wird somit durch die Analyse durch Messung bzw. Wahrnehmung eines Benutzers herausgefunden, ob die zu dem wenigstens einen Antigen zugehörigen Antikörper in der Probe vorhanden sind bzw. oberhalb der Nachweisgrenze des Verfahrens liegen oder nicht vorhanden sind bzw. unterhalb der Nachweisgrenze des Verfahrens liegen.

Indem der erfindungsgemäße Schnelltest lediglich eine Entnahme einer Probe von Körperflüssigkeit erfordert, welche dann einem Reagenz zugeführt wird und anschließend das Gemisch aus Reagenz und Probe einer Analyse unterzogen wird, ist ein Ergebnis bereits mit wenigen einfachen Eingriffen von der Probenahme bis zum Testergebnis erbringbar. Auch sind die Ergebnisse der Untersuchung zeitnah verfügbar. Damit eignet sich der erfindungsgemäße Schnelltest besonders zur Überprüfung der Wirksamkeit einer Impfung gegen HPV, beispielsweise in der Praxis des impfenden Arztes.

Die von dem Benutzer wahrnehmbare Veränderung im Zuge der Analyse kann eine optische, akustische und/oder taktile Veränderung sein. Beispielsweise kann wenigstens ein fluoreszierendes Medium genutzt werden. Die Analyse kann einen Färbungseffekt bzw. Verfärbungseffekt oder eine Farbreaktion nutzen. Grundsätzlich kann die Analyse jegliche Art einer messbaren Reaktion nutzen. Durch alle diese Möglichkeiten kann das Analyseergebnis rasch ermittelt werden.

Der erfindungsgemäße Schnelltest ist zum Nachweis von Antikörpern gegen humane Papillomviren geeignet. Derartige Viren wirken infizierend auf Menschen. Grundsätzlich kann der erfindungsgemäße Schnelltest auch zum Nachweis von Antikörpern gegen nicht-humane Papillomviren eingesetzt werden, welche beispielsweise Tiere infizieren. In diesem Fall ist ein entsprechend anderes Antigen im Zuge des Schnelltestes zu verwenden.

Bevorzugt wird bei fehlenden HPV-Antikörpern in der Probe oder bei einer Menge von HPV-Antikörpern in der Probe, welche unterhalb oder im Bereich einer vorgegebenen Nachweisgrenze für das HPV-spezifische Antigen liegen, ein Medium von einem Benutzer wahrnehmbar und/oder messbar verändert. Die Veränderung kann über einen vorgegebenen Flächenabschnitt des Mediums oder über ein vorgegebenes Volumen des Mediums stattfinden.

Es sollte der Analyse eine vorgegebene Menge des Gemisches aus dem Reagenz und der Probe an Körperflüssigkeit zugeführt werden. Bevorzugt sollte die vorgegebene Menge eine Teilmenge des vorhandenen Gemisches sein. Dadurch lassen sich aus einer entnommenen Probe und dem damit vereinigten Reagenz mehrere diagnostische Untersuchungen vornehmen, beispielsweise um eine statistische Absicherung des Ergebnisses zu erlangen.

Es kann ferner die Menge des wenigstens einen HPV-spezifischen Antigens durch eine vorgegebene Nachweisgrenze für die zu bestimmende Menge des in der Flüssigkeitsprobe enthaltenen wenigstens einen HPV-Antikörpers bestimmt werden. Die Nachweisgrenze ist bevorzugt die jeweils erwünschte Nachweisgrenze, beispielsweise um zu ermitteln, dass eine bestimmte Menge an Antikörpern gegen HPV in der Flüssigkeitsprobe ist, um damit die Immunität, beispielsweise aufgrund einer erfolgreichen Impfung, zu bestätigen.

Es wird wenigstens ein in der Probe enthaltener Typ von Antikörpern gegen HPV durch die Auswahl des wenigstens einen eingesetzten HPV-spezifischen Antigens qualitativ bestimmt. Es wird ferner durch eine entsprechende Auswahl des Antigens bestimmt, ob in der Probe Antikörper gegen humane Papillomviren des Typs 16 enthalten sind. Dazu wird ein HPV16-spezifisches Antigen bei dem Schnelltest eingesetzt.

Weiterhin wird durch eine entsprechende Auswahl des Antigens bestimmt, ob in der Probe Antikörper gegen humane Papillomviren mit dem L1-Protein enthalten sind, nämlich ob es sich um das L1-Protein der HPV vom Typ 16 handelt. Dazu wird ein HPV16L1-spezifisches Antigen bei dem Schnelltest eingesetzt.

Im Zuge der Erfindung ist unter dem HPV16 zu verstehen, dass es sich um den Typ 16 der humanen Papillomviren handelt. Ferner ist im Zuge der Erfindung unter HPV16L1 zu verstehen, dass es sich um das als L1 bezeichnete Protein der humanen Papillomviren des Typs 16 handelt. Bei dem zum Einsatz kommenden Antigen handelt es sich im Zuge der Erfindung um Virusbestandteile, die eine Immunantwort auslösen können.

Nach einem weiteren Aspekt betrifft die Erfindung eine Vorrichtung zur Durchführung eines Schnelltests zum qualitativen und/oder quantitativen Bestimmen von in Körperflüssigkeit enthaltenen Antikörpern gegen humane Papillomviren. Die erfindungsgemäße Vorrichtung kann für einen Schnelltest der vorstehend beschriebenen Art eingesetzt werden.

Die Vorrichtung zur Durchführung eines Schnelltests zum qualitativen und/oder quantitativen Bestimmen von in Körperflüssigkeit enthaltenen Antikörpern gegen humane Papillomviren weist ein Behältnis auf, in welchem ein Reagenz im Wesentlichen aus einer vorgegebenen Menge an physiologisch wirkender Flüssigkeit, wie beispielsweise Kochsalzlösung, und einer vorgegebenen Menge wenigstens eines HPV-spezifischen Antigens enthalten ist, wobei zu dem Reagenz Körperflüssigkeit aus einer Patientenprobe in den Behälter einfüllbar ist.

Die Vorrichtung weist ferner ein Analysesystem auf, welches eine messbare und/oder von einem Benutzer wahrnehmbare Veränderung nutzt. Beispielsweise kann das Analysesystem einen Färbungseffekt bzw. Verfärbungseffekt oder eine Farbreaktion nutzen. Das Analysesystem ist ferner zur Aufnahme einer vorgegebenen Menge eines in dem Behältnis befindlichen Substrates ausgebildet ist.

Die erfindungsgemäße Vorrichtung ist dadurch einfach aufgebaut und ermöglicht mit geringem Aufwand eine diagnostische Untersuchung der Patientenprobe bereits vor Ort, beispielsweise am Ort der Probenahme. Es ist damit die Bestimmung von in Körperflüssigkeit enthaltenen Antikörpern gegen humane Papillomviren bereits in der Praxis eines niedergelassenen Arztes oder im Krankenhaus unmittelbar auf der Krankenstation möglich. Eine aufwendige und teure Analyse in einem Zentrallabor ist nicht notwendig. Bereits dadurch ergibt sich ein zeitlicher Vorteil bei einer mit der erfindungsgemäßen Vorrichtung durchgeführten Untersuchung.

Indem das Analysesystem einen Färbungseffekt oder eine Farbreaktion nutzen kann, kann auf den Einsatz handelsüblicher und besonders kostengünstiger Teststreifen zurückgegriffen werden, wie sie beispielsweise in Kassettenform angeboten werden. Um ein derartiges Analysesystem einsetzen zu können, wird bei der erfindungsgemäßen Vorrichtung das sich an die Antikörper bindende wenigstens eine HPV-spezifische Antigen in dem Behälter vorgehalten. Die physiologisch wirkende Flüssigkeit in dem Behälter dient dazu, das Antigen intakt zu halten und somit für einen vorbestimmten Zeitraum den Einsatz der Vorrichtung mit einer solchen biologischen Komponente zu ermöglichen. Ein etwaiges Trocknen der Flüssigkeit würde beispielsweise das Antigen zerstören.

Als Antigen kann jedes beliebige Antigen genutzt werden, welches sich an Antikörper gegen Papillomviren bindet. Da Antikörper gegen humane Papillomviren des Typs 16L1 nachgewiesen werden sollen, sind entsprechende HPV16L1-spezifische Antigene einzusetzen. Es werden dabei aggregierte Antigene benutzt.

Die erfindungsgemäße Vorrichtung ermöglicht eine diagnostische Untersuchung mit einer Probe, welche eine Körperflüssigkeit beliebiger Art enthält. Als Körperflüssigkeit kann beispielsweise Blut, insbesondere Vollblut, Serum, Plasma, Speichel und Sekret verwendet werden.

Es bietet sich an, dass das Behältnis separat ist. Dadurch bildet das Behältnis eine separate Einheit gegenüber dem Analysesystem, welches wiederum eine separate Einheit darstellt. Der Behälter kann dadurch getrennt und unabhängig von dem Analysesystem genutzt werden.

Es bietet sich ferner an, dass das Behältnis verschließbar ist. Dazu kann das Behältnis einen Deckel aufweisen, welcher beispielsweise mittels eines Gewindes auf den Behälter aufschraubbar ist. Indem das Behältnis abschließbar ist, kann in einfacher Weise das Reagenz und/oder die Probe an Körperflüssigkeit eingebracht werden. Durch das Verschließen des Behältnisses ist eine Leckage freie Aufbewahrung sowie Handhabung des Behältnisses ermöglicht. Beispielsweise kann in dem verschlossenen Zustand des Behältnisses besonders gut ein Vermischen von Patientenprobe und Reagenz erzielt werden, indem das geschlossene Behältnis durch einen Benutzer hin und her geschüttelt wird.

Weiterhin bietet es sich an, dass das Behältnis und gegebenenfalls ein Deckel oder eine Verschlusskappe aus Kunststoff sind. Dadurch ist das Behältnis besonders kostengünstig herstellbar.

Nach einer Ausgestaltung der Erfindung kann das Behältnis ein Innengefäß und ein das Innengefäß umgebendes Gehäuse aufweisen. Das Innengefäß kann zylindrisch oder konisch ausgebildet sein. Durch das Gehäuse lässt sich das Behältnis gut handhaben, insbesondere gut ergreifen. Auch bietet das Gehäuse einen Schutz auf das Innengefäß gegen Einwirkungen von außen.

Nach einer weiteren Ausgestaltung kann das Behältnis eine Entnahme bzw. Aufnahme für die Patientenprobe, insbesondere eine kapillare Entnahme bzw. Aufnahme, aufweisen. Dadurch kann beispielsweise eine Blutmenge bereits mittels dem Behältnis selbst über die Entnahme bzw. kapillare Entnahme in einfacher Weise aufgenommen und in den Innenraum geführt werden, in welchem sich das Reagenz befindet.

Der Schnelltest von der Entnahme der Probe bis hin zu dem Vereinigen und Mischen mit dem das Antigen enthaltenen Reagenz ist damit besonders einfach und mit besonders wenig Aufwand durchführbar.

Nach der Erfindung ist es vorgesehen, dass das Analysesystem ein Konjugat aufweist, welches im Wesentlichen aus einer vorgegebenen Menge wenigstens eines HPV-spezifischen Moleküls und einem Stoff besteht, der eine messbare und/oder von einem Benutzer wahrnehmbare Reaktion ergibt. Beispielsweise kann der Stoff eine Farbreaktion bewirken oder bei räumlicher Häufung eine Färbung ergeben.

Durch den Stoff ist eine farbliche Markierung der zu bestimmenden Komponenten möglich. Durch das HPV-spezifische Molekül wird eine Reaktion mit einem zugehörigen noch reaktiven HPV-spezifischen Antigen stattfinden, so dass dadurch über das Vorhandensein des reaktiven HPV-spezifischen Antigens ein Farbnachweis im Zuge der diagnostischen Untersuchung stattfindet.

Das HPV-spezifische Molekül des Konjugats ist ein HPV16L1 spezifisches Molekül. Ein solches Molekül kann ein Antikörper sein. Denkbar ist es ferner, dass das HPV-spezifische Molekül ein zelluläres Molekül der humanen Zelle ist, das als Rezeptor fungiert. Maßgeblich ist, dass das Molekül mit dem Virusanteil des Substrates aus dem Behältnis, also dem HPV-Reagenz, reagiert, sofern dieser Anteil noch reaktiv ist. Die Reaktion bewirkt eine Anbindung des Moleküls an freie Bindungsstellen des HPV-Reagenz bzw. des Antigens.

Der zum Markieren genutzte Stoff besteht vorzugsweise aus kolloidalem Gold und/oder Silber. Die Goldpartikel bzw. Silberpartikel des Stoffes weisen dabei vorzugsweise einen Durchmesser von etwa 2 nm (Nanometer) bis etwa 100 nm (Nanometer) auf. Bevorzugt liegt das kolloidale Gold bzw. Silber in aggregierter Form vor.

Nach der Erfindung weist das Analysesystem ein Trägermedium auf, an oder in dem die aufgegebene Menge des Substrates aus dem Behältnis, vorzugsweise ausgehend von einer Befüllstelle, in Richtung zumindest einer Raumachse fließbar ist. Dadurch kann der von dem Substrat zurückgelegte Weg auf dem Trägermedium genutzt werden, um an wenigstens einer vorgegebenen Stelle des Weges wenigstens ein Farbsignal zu geben. Es ist dadurch eine Klassifizierung nach verschiedenen Farbsignalen möglich.

Es bietet sich an, dass das Trägermedium ein poröses Medium, insbesondere eine poröse Membran, ist. Dadurch wird das Fließen des Substrates in Richtung der wenigstens einen Raumachse begünstigt.

Es bietet sich ferner an, dass ein Medium vorgesehen ist, welches den Fluss des Substrates erzeugt. Das Medium kann am Ende des Fließweges des Substrates liegen. Das Medium kann durch das Trägermedium selbst gebildet sein, beispielsweise wenn das Trägermedium eine poröse Membran ist. Das Medium kann dann mehrlagig durch einen Abschnitt des Trägermediums gebildet sein, so dass in diesem Bereich das Trägermedium einen höheren Saugeffekt hat als in dem wenigstens einen anderen Abschnitt, welcher durch weniger Lagen bzw. Schichten gebildet ist und damit geringere Saugeigenschaften aufweist.

Das Trägermedium und/oder Medium zum Erzeugen des Flusses kann durch eine Schaumstoffmatte, ein Löschpapier oder ein Fließpapier oder eine Kombination davon gebildet sein.

Nach der Erfindung weist das Analysesystem eine vorgegebene Menge wenigstens eines Moleküls auf, welches an einer vorgegebenen ersten Stelle oder einem vorgegebenen ersten Bereich des Trägermediums fixiert ist und bei reaktivem Reagenz des Behälters sich an das Antigen bindet. Dadurch werden diejenigen Moleküle des Substrates festgehalten, welche in Reaktion mit dem Konjugat gehen und damit eine Verbindung mit dem Konjugat eingehen. Das wenigstens eine an dem Trägermedium fixierte Molekül wirkt dabei als Fangmolekül, so dass die mit dem Konjugat in Reaktion gegangenen Komponenten des Substrates an den vorgegebenen ersten Bereich bzw. der ersten Stelle festgehalten werden.

Es ist ferner vorgesehen, dass das wenigstens eine Molekül, welches auf dem Trägermedium fixiert ist und das wenigstens eine Molekül, welches das Konjugat bildet, miteinander identisch sind. Selbstverständlich können auch zueinander unterschiedliche Moleküle eingesetzt werden. Maßgeblich ist es dabei, dass beide Moleküle, sowohl das Fangmolekül als auch das Konjugat-Molekül, eine Bindung mit dem entsprechenden Anteil des Substrates, also dem humanen Anteil oder dem Virusanteil, eingehen.

Eine weitere Ausgestaltung der Erfindung sieht vor, dass das Analysesystem eine vorgegebene Menge wenigstens eines Moleküls aufweist, welche sich an wenigstens einem Bestandteil des Konjugats bindet und welches an einer vorgegebenen zweiten Stelle oder einem vorgegebenen zweiten Bereich des Trägermediums fixiert ist, wobei in Fließrichtung des aufgegebenen Substrates gesehen, die zweite Stelle oder der zweite Bereich nach der ersten Stelle angeordnet ist. Dadurch ist eine Kontrolle durchführbar, ob das auf das Trägermedium bzw. in das Trägermedium gegebene Substrat bis zu der zweiten Stelle bzw. dem zweiten Bereich geflossen ist. Sofern ein Farbsignal am zweiten Bereich bzw. der zweiten Stelle erfolgt, ist damit sichergestellt, dass das Konjugat das Substrat zumindest mit seinen mit dem Konjugat verbundenen Komponenten die erste Stelle bzw. den ersten Bereich auch tatsächlich passiert und damit die aufgegebene Menge an Substrat auf das Analysesystem ausreichend war.

Nach einem weiteren Aspekt umfasst die Erfindung ein Behältnis der vorstehend beschriebenen Art.

Auch umfasst die Erfindung ein Analysesystem der vorstehend beschriebenen Art.

Die Erfindung ermöglicht es, dass belastbare Testergebnisse bereits innerhalb von weniger als einer Stunde nach der Entnahme einer Probe von Körperflüssigkeit vorliegen. Es hat sich nämlich gezeigt, dass bereits nach wenigen Minuten der Zuführung der Probe von Körperflüssigkeit zu dem Reagenz mit dem HPV-spezifischen Antigen es zu der angestrebten Reaktion des Gemisches kommt und somit bereits nach dieser kurzen Zeit die Analyse des Gemisches vorgenommen werden kann. Sofern zur Analyse Teststreifen genutzt werden, hat es sich gezeigt, dass der Schnelltest innerhalb von 15 bis etwa 25 Minuten ab der Probenahme durchgeführt werden kann und dann bereits ein belastbares Ergebnis vorliegt.

### Ausführungsbeispiel

Weitere Ziele, Vorteile, Merkmale und Anwendungsmöglichkeiten der vorliegenden Erfindung ergeben sich aus der nachfolgenden Beschreibung eines Ausführungsbeispieles anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Fig. 1: eine mögliche Ausführungsform eines Behältnisses einer Vorrichtung zur Durchführung eines Schnelltests zum qualitativen und/oder quantitativen Bestimmen von in Körperflüssigkeit enthaltenen Antikörpern gegen humane Papillomviren in Seitenansicht,
- Fig.2: eine mögliche Ausführungsform eines Analysensystems einer Vorrichtung zur Durchführung eines Schnelltests zum qualitativen und/oder quantitativen Bestimmen von in Körperflüssigkeit enthaltenen Antikörpern gegen humane Papillomviren in Draufsicht,
- Fig. 3: das Analysesystem gemäß der Figur 2 nach Durchführung eines Schnelltests zum qualitativen und/oder quantitativen Bestimmen von in Körperflüssigkeit enthaltenen Antikörpern gegen humane Papillomviren mit negativem Ergebnis,
- Fig. 4: das Analysesystem gemäß der Figur 2 nach Durchführung eines Schnelltests zum qualitativen und/oder quantitativen Bestimmen von in Körperflüssigkeit enthaltenen Antikörpern gegen humane Papillomviren mit positivem Ergebnis und
- Fig. 5A bis 5F: Verfahrensschritte bei der Durchführung eines Schnelltests zum qualitativen und/oder quantitativen Bestimmen von in Körperflüssigkeit enthaltenen Antikörpern gegen humane Papillomviren.

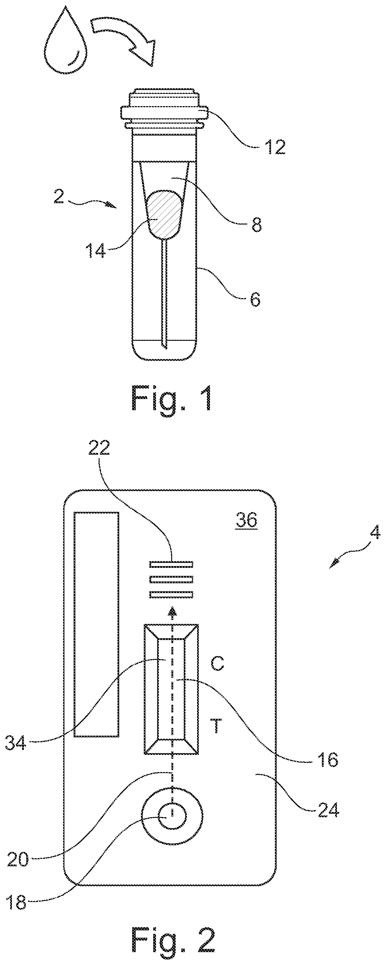

Figuren 1 und 2 zeigen - in schematischer Darstellung - eine mögliche Ausführungsform einer Vorrichtung zur Durchführung eines Schnelltests zum qualitativen und/oder quantitativen Bestimmen von in Körperflüssigkeit enthaltenen Antikörpern gegen humane Papillomviren, welche wenigstens ein Behältnis 2 (Figur 1) und ein Analysesystem 4 (Figur 2) umfasst. Die Vorrichtung ermöglicht die Durchführung des Schnelltests an Proben beliebiger Körperflüssigkeit, wie beispielsweise Vollblut, Serum, Plasma, Speichel und Sekret.

Mittels der Vorrichtung können in der Probe enthaltene humane Antikörper gegen HPV beliebigen Typs und grundsätzlich auch beliebiger Konzentration bestimmt werden. Der Einfachheit halber beziehen sich die nachfolgenden Ausführungen beispielhaft auf die Ausführungsform der Vorrichtung, mittels welcher in der Probe enthaltene Antikörper vom Typ 16L1 nachgewiesen werden. Die nachfolgend beschriebene Ausführungsform ist ferner darauf ausgelegt, dass bei einem positiven Analyseergebnis eine so hohe Konzentration an HPV16L1 Antikörpern in der Patientenprobe enthalten sind, dass die beprobte Person sicher gegen humane Papillomviren immunisiert ist.

Das Behältnis 2 weist in seinem Inneren ein Reagenz 14 auf, welches im Wesentlichen aus einer vorgegebenen Menge an physiologisch wirkender Flüssigkeit, wie beispielsweise einer Kochsalzlösung, und einer vorgegebenen Menge eines HPV16L1-spezifischen Antigens besteht. Das Behältnis 2 ist bevorzugt in Art eines Röhrchens ausgebildet, dessen Außenumfang ein Gehäuse 6 bildet. Das Gehäuse 6 umgibt ein Innengefäß 8, in welchem sich das Reagenz 14 befindet. Das Innengefäß 8 kann konisch ausgebildet sein.

Das Behältnis 2 weist ferner eine Verschlusskappe 12 auf, welche vorzugsweise auf eine Öffnung des Behältnisses 2 aufbringbar, insbesondere aufschraubbar ist. Über die von der Verschlusskappe 12 abdeckbare Öffnung des Behältnisses 2 ist selbstverständlich auch Körperflüssigkeit aus der Patientenprobe dem Inneren des Behältnisses 2 zuzuführen, indem sich das Reagenz 14 befindet.

Das innere des Behältnisses 2 bzw. das Innengefäß 8 bildet einen im Wesentlichen Leckage freien Innenraum, welcher selbst bei Schütteln des Behältnisses 2 im Wesentlichen Leckage frei ist.

Das Analysesystem 4 führt die diagnostische Untersuchung aus, indem ein Färbungseffekt bzw. ein Verfärbungseffekt genutzt wird. Damit ist durch das Analysesystem 4 ein Analyseergebnis zeitnah verfügbar und die Analyse kann bereits vor Ort in der Praxis des behandelnden Arztes oder im Krankenhaus unmittelbar auf der Krankenstation vorgenommen werden.

Das Analysesystem 4 weist eine Befüllstelle 18 auf, in welcher eine vorgegebene Menge an zu analysierendem Substrat eingefüllt werden kann. Die einfüllbare Menge an Substrat wird dabei durch die Tiefe und vorzugsweise dem Durchmesser der Öffnungsberandung bzw. der Umfangswandung der Befüllstelle 18 vorgegeben.

Die Befüllstelle 18 ist beispielsweise durch eine Durchgangsöffnung durch ein Deckelteil 36 eines Gehäuses 24 gebildet. Zusätzlich zu dem Deckelteil 36 weist das Gehäuse 24 ein (in der Figur 2 nicht sichtbares) Bodenteil auf, wobei zwischen dem Bodenteil und dem Deckelteil 36 ein Trägermedium 16 angeordnet ist, welches vorzugsweise aus einem porösen Material, insbesondere einer porösen Membran, gebildet ist.

Das Deckelteil 36 und das (nicht sichtbare) Bodenteil können gegeneinander angeformt sein, so dass das Gehäuse einteilig ausgebildet ist. Selbstverständlich können Deckelteil 36 und das (nicht sichtbare) Bodenteil wenigstens zwei separate Teile sein.

Das Trägermedium 16 bzw. die poröse Membran ist länglich ausgebildet, und überragt die Öffnung der Befüllstelle 18 zumindest teilweise, so dass ein in die Befüllstelle 18 eingefülltes bzw. eingetropftes Substrat in Richtung gemäß dem Pfeil 20 fließen kann. Das Fließen des Substrates wird durch ein Medium 22 hervorgerufen, welches besonders saugfähig ist und dadurch einen Fluss des Substrates in Richtung gemäß dem Pfeil 20 erzeugt. Das Medium 22 ist bei der Ausführungsform des Analysesystems 4 gemäß der Figur 2 durch mehrlagig übereinander gelegte Abschnitte des Trägermediums 16 gebildet, wodurch in diesem Bereich das Trägermedium eine größere Saugfähigkeit aufweist, als in dem weniger lagigen, insbesondere einlagigen Bereich, welcher zwischen der Befüllstelle 18 und dem Medium 22 ausgebildet ist.

Das Analysensystem 4 weist ferner ein (in der Figur 2 nicht sichtbares) Konjugat auf, welches im Wesentlichen aus einer vorgegebenen Menge eines HPV16L1-spezifischen Moleküls besteht, welches mit kolloidalem Gold markiert ist.

Bevorzugt ist das (in der Figur 2 nicht sichtbare) Konjugat in der Nähe oder im Bereich der Befüllstelle 18 auf dem Trägermedium 16 angeordnet, so dass bei Befüllen des Analysesystems 4 über die Befüllstelle 18 das Substrat direkt mit dem Konjugat in Verbindung tritt.

Weiterhin weist das Analysesystem 4 eine vorgegebene Menge eines (in der Figur 2 nicht sichtbaren) Moleküls auf, welches an einen vorgegebenen ersten Bereich an dem Trägermedium 16 fixiert ist. Der (in der Figur 2 nicht sichtbare) erste Bereich ist in Fließrichtung 20 gesehen, etwa im Bereich der auf dem Deckelteil 36 vorgesehenen Kennzeichnung T angeordnet.

Weiterhin weist das Analysesystem 4 eine vorgegebene Menge eines (in der Figur 2 nicht sichtbaren) Moleküls auf, welches an einem vorgegebenen zweiten Bereich an dem Trägermedium 16 fixiert ist. Der zweite Bereich ist in Fließrichtung 20 gesehen, etwa im Bereich der auf dem Deckelteil 36 vorgesehenen Kennzeichnung C angeordnet und ist somit dem ersten Bereich nachgeschaltet.

Die in dem ersten Bereich fixierte Menge des einen (in der Figur 2 nicht sichtbaren) Moleküls hat die Eigenschaft, bei einem reaktiven Substrat, welches in Fließrichtung 20 fließt und den ersten Bereich erreicht, mit einem Bestandteil des Substrates sich zu verbinden. Dem Molekül kommt damit eine Fangwirkung im Hinblick auf das noch reaktive Substrat zu, welches im Falle einer Bindung über das Konjugat sich in dem ersten Bereich ansammelt.

Die vorgegebene Menge des wenigstens einen anderen Moleküls ist vorzugsweise ebenfalls Konjugat spezifisch, so dass in Richtung des Pfeiles 20 fließendes Substrat mitsamt dem Konjugat bei Erreichen des zweiten Bereiches von den dort vorhandenen fixierten Molekülen gebunden wird und es somit auch an diesem zweiten Bereich zu einer Anhäufung des Konjugats kommt.

Aufgrund der Markierung des Konjugats mit kolloidalem Gold wird bewirkt die Anreicherung in dem ersten Bereich bzw. dem zweiten Bereich jeweils eine sichtbare Verfärbung auf dem Trägermedium 16. Damit diese Verfärbung auch von dem Benutzer des Analysesystems 4 optisch wahrgenommen werden kann, weist das Deckelteil 36 eine Durchgangsöffnung auf, welche ein Sichtfeld 34 bildet, über welches der Benutzer auf das Trägermedium 16 sehen kann.

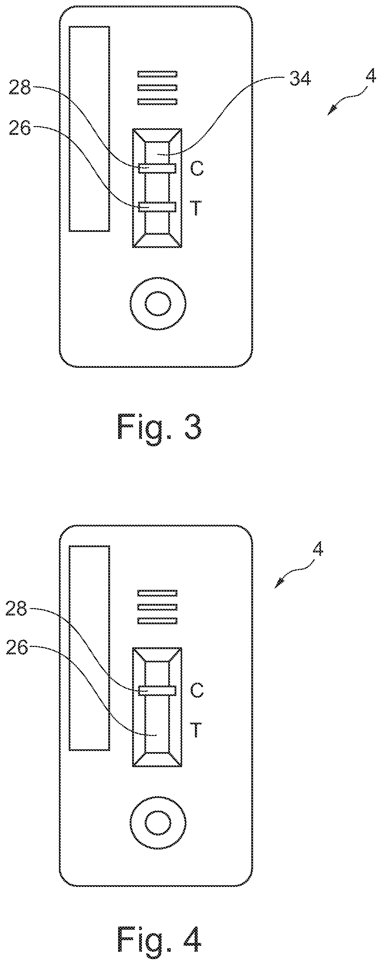

Figur 3 zeigt das Analysesystem 4 nach Durchführung eines Schnelltests mit einer Patientenprobe, welche keine humanen Antikörper gegen HPV16L1 enthält. Wie daraus ersichtlich ist, ist im ersten Bereich 26 sowie im zweiten Bereich 28 jeweils eine verfärbte Linie in dem Sichtfeld 34 sichtbar.

Da in der entnommenen Patientenprobe keine Antikörper gegen HPV16L1 vorliegen, ist das Reagenz des Behältnisses 2 beim Vermischen mit der Patientenprobe reaktiv geblieben. Das aus dem Behältnis 2 entnommene Substrat aus der Patientenprobe und dem Reagenz ist dadurch auch noch reaktiv im Hinblick auf das Analysesystem 4. Damit bindet das Substrat in diesem Fall an das Konjugat auf dem Trägermedium 16. Der dadurch entstandene Komplex ist von der Menge an Molekülen in dem ersten Bereich 26 gebunden worden und durch die Menge des einen im ersten Bereich 26 fixierten Moleküls dort auf dem Trägermedium 16 festgehalten worden, so dass aufgrund des kolloidalen Goldes im Konjugat eine optische Verfärbung im ersten Bereich 26 stattgefunden hat.

Der Komplex aus dem Substrat, zumindest das Konjugat, ist weiter in Richtung gemäß Pfeil 20 bis zu dem zweiten Bereich 28 geflossen. Die in dem zweiten Bereich 28 an dem Trägermedium 16 fixierte Menge des wenigstens einen anderen Moleküls hat den Komplex und damit das kolloidale Gold auch im zweiten Bereich 28 gefangen und aufgrund der Häufung des kolloidalen Goldes zu einer optischen Verfärbung im zweiten Bereich 28 geführt.

Durch die Verfärbung des ersten Bereiches 26 ist nachgewiesen, dass keine Antikörper gegen HPV16L1 oder lediglich Antikörper unter der Nachweisgrenze in der Patientenprobe sind. Ferner ist durch die Verfärbung in dem zweiten Bereich 28 bestätigt, dass Substrat und Konjugat bis in den zweiten Bereich 28 geflossen sind und damit die in die Befüllstelle 18 eingefüllte Probenmenge ausreichend für die Analyse war.

Figur 4 zeigt das Ergebnis eines Schnelltests mit einer Patientenprobe, welche humane Antikörper gegen HPV16L1 in einer über der Nachweisgrenze des Analysensystems 4 liegenden Menge enthält. In dem ersten Bereich 26 ist eine Verfärbung ausgeblieben. Dagegen weist der zweite Bereich 28 eine Verfärbung auf, so dass dadurch die ordnungsgemäße Aufgabe einer ausreichenden Substratmenge in die Befüllstelle 18 bestätigt ist. Die ausbleibende Verfärbung im ersten Bereich 26 ist darauf zurückzuführen, dass das in dem Behältnis 2 enthaltene Reagenz 14 durch die in der Patientenprobe enthaltenen HPV16L1 Antikörper inaktiviert wurde. Dadurch findet in dem Analysesystem 4 eine Reaktion des Substrates mit dem Konjugat nicht statt, so dass im ersten Bereich 26 ein Farbsignal ausbleibt.

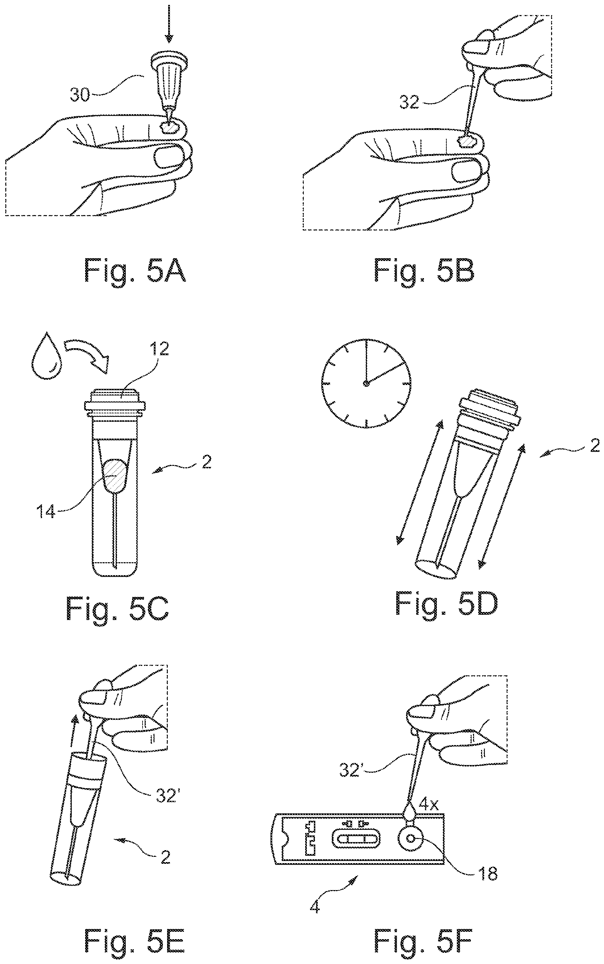

Figuren 5A bis 5F zeigen die Verfahrensschritte bei der Durchführung eines Schnelltests zum qualitativen bzw. quantitativen Bestimmen von in Körperflüssigkeit enthaltenen Antikörper gegen humane Papillomviren, bei dem das Behältnis 2 und das Analysesystem 4 zum Einsatz kommt.

Es wird eine Probe an Vollblut entnommen. Dazu kann beispielsweise mittels einer Lanzette 30 ein Stich in den Finger durchgeführt werden und daraus eine vorgegebene Menge an Blut mittels einer Pipette 32 aufgenommen werden (Figuren 5A und 5B). Anschließend wird eine vorgegebene Menge des Blutes dem Behältnis 2 mit dem Reagenz 14 zugeführt und das Behältnis 2 mehrfach geschüttelt und über einen vorgegebenen Zeitraum, beispielsweise 10 Minuten, stehen gelassen. Hierdurch hat eine Vermischung der Probenmenge mit dem Reagenz 14 stattgefunden (Figur 5D).

In einem weiteren Schritt wird dann das in dem Behältnis 2 nun befindliche Substrat mittels einer Pipette 32' zumindest teilweise entnommen und eine vorgegebene Menge, beispielsweise einer oder mehrere Tropfen des Substrates, über die Befüllstelle 18 dem Analysesystem 4 zugeführt. Je nachdem, ob in der Patientenprobe eine über der Nachweisgrenze des Analysesystems 4 liegende Menge an Antikörpern gegen HPV16L1 vorhanden sind oder nicht, kommt es zur Verfärbung im ersten Bereich 26 oder es bleibt eine solche Verfärbung aus (Figur 5F).

### Bezugszeichenliste

- 2: Behältnis
- 4: Analysesystem
- 6: Gehäuse
- 8: Innengefäß
- 12: Verschlusskappe
- 14: Reagenz
- 16: Trägermedium
- 18: Befüllstelle
- 20: Fließrichtung
- 22: Medium
- 24: Gehäuse
- 26: erster Bereich
- 28: zweiter Bereich
- 30: Lanzette
- 32, 32': Pipette
- 34: Sichtfeld
- 36: Deckelteil

## Patentansprüche

1. Schnelltest zum qualitativen und/oder quantitativen Bestimmen von in Körperflüssigkeit enthaltenen Antikörpern gegen humane Papillomviren vom Typ 16 (HPV16),
bei dem eine Probe von Körperflüssigkeit mit einem Reagenz (14) gemischt wird, welches im Wesentlichen aus einer vorgegebenen Menge an physiologisch wirkender Flüssigkeit und einer vorgegebenen Menge wenigstens eines Antigens besteht, wobei das Antigen aggregiert ist und bezüglich in der Körperflüssigkeit enthaltener Antikörper reaktiv ist, welche gegen das L1-Protein der HPV16 spezifisch sind (HPV16L1 spezifische Antikörper) und wobei die Flüssigkeit auf das Antigen physiologisch wirkend ist, um die Reaktivität des Antigens in Bezug auf diese Antikörper aufrecht zu erhalten, und
bei dem anschließend das Gemisch einer Analyse zugeführt wird, die Analyse nutzend:
• ein Konjugat, welches im Wesentlichen aus einer vorgegebenen Menge wenigstens eines Moleküls und einem Stoff besteht, der eine messbare und/oder von einem Benutzer wahrnehmbare Reaktion bewirkt oder bei räumlicher Häufung eine messbare und/oder von einem Benutzer wahrnehmbare Veränderung ergibt, wobei das wenigstens eine Molekül gegen das L1-Protein der HPV16 spezifisch ist und sich an das Antigen bindet, wenn das Antigen noch reaktiv ist,
• ein Trägermedium (16), an oder in dem die aufgegebene Menge des Gemisches in Richtung zumindest einer Raumachse (20) fließbar ist,
• eine vorgegebene Menge wenigstens eines Fangmoleküls, welches an einer vorgegebenen ersten Stelle oder einem vorgegebenen ersten Bereich (26) des Trägermediums (16) fixiert ist und sich an das wenigstens eine Antigen bindet, wenn das Antigen noch reaktiv ist,
wobei das Fangmolekül und das Molekül des Konjugats derart zueinander identisch sind, dass beide eine Bindung mit dem Antigen eingehen, und das Antigen noch reaktiv ist, wenn in der Probe von Körperflüssigkeit HPV16L1 spezifische Antikörper nicht vorhanden sind oder unterhalb der Nachweisgrenze des Verfahrens liegen.

2. Schnelltest nach Anspruch 1, **dadurch gekennzeichnet, dass** eine vorgegebene Menge des Gemisches der Analyse zugeführt wird.

3. Schnelltest nach Anspruch 1 oder 2, **dadurch gekennzeichnet, dass** die Menge des wenigstens einen Antigens durch eine vorgegebene Nachweisgrenze für die zu bestimmende Menge der in der Flüssigkeitsprobe enthaltenen HPV16L1 spezifischen Antikörper bestimmt wird.

4. Schnelltest nach einem der vorhergehenden Ansprüche,
**dadurch gekennzeichnet, dass** bei fehlenden HPV16L1 spezifischen Antikörpern in der Probe oder bei einer Menge von HPV16L1 spezifischen Antikörpern in der Probe, welche unterhalb oder im Bereich einer vorgegebenen Nachweisgrenze für die HPV16L1 spezifischen Antikörper liegen, ein Medium, insbesondere über einen vorgegebenen Flächenabschnitt oder ein vorgegebenes Volumen, von einem Benutzer wahrnehmbar und/oder messbar verändert wird.

5. Vorrichtung zur Durchführung eines Schnelltests zum qualitativen oder quantitativen Bestimmen von in Körperflüssigkeit enthaltenen Antikörpern gegen humane Papillomviren vom Typ 16 (HPV16), insbesondere nach einem der vorhergehenden Ansprüche,
mit einem Behältnis (2), in welchem ein Reagenz (14) im Wesentlichen aus einer vorgegebenen Menge an physiologisch wirkender Flüssigkeit und einer vorgegebenen Menge wenigstens eines Antigens enthalten ist und in welchen zu dem Reagenz (14) Körperflüssigkeit aus einer Patientenprobe einfüllbar ist, wobei das Antigen aggregiert ist und bezüglich in der Körperflüssigkeit enthaltener Antikörper reaktiv ist, welche gegen das L1-Protein der HPV16 spezifisch sind (HPV16L1 spezifische Antikörper), und wobei die Flüssigkeit auf das Antigen physiologisch wirkend ist, um die Reaktivität des Antigens in Bezug auf diese Antikörper aufrecht zu erhalten, und
mit einem Analysesystem (4), welches zur Aufnahme einer vorgegebenen Menge eines in dem Behältnis (2) befindlichen Substrates aus dem Reagenz (14) und der Patientenprobe ausgebildet ist und eine durch das Substrat bewirkte messbare und/oder von einem Benutzer wahrnehmbare Veränderung nutzt, das Analysesystem (4) aufweisend:
• ein Konjugat, welches im Wesentlichen aus einer vorgegebenen Menge wenigstens eines Moleküls und einem Stoff besteht, der eine messbare und/oder von einem Benutzer wahrnehmbare Reaktion bewirkt oder bei räumlicher Häufung eine messbare und/oder von einem Benutzer wahrnehmbare Veränderung ergibt, wobei das wenigstens eine Molekül gegen das L1-Protein der HPV16 spezifisch ist und sich an das Antigen bindet, wenn das Antigen noch reaktiv ist,
• ein Trägermedium (16), an oder in dem die aufgegebene Menge des Substrates aus dem Behältnis (2) in Richtung zumindest einer Raumachse (20) fließbar ist,
• eine vorgegebene Menge wenigstens eines Fangmoleküls, welches an einer vorgegebenen ersten Stelle oder einem vorgegebenen ersten Bereich (26) des Trägermediums (16) fixiert ist und sich an das wenigstens eine Antigen bindet, wenn das Antigen noch reaktiv ist,
wobei das Fangmolekül und das Molekül des Konjugats derart zueinander identisch sind, dass beide eine Bindung mit dem Antigen eingehen, und das Antigen noch reaktiv ist, wenn in der Probe von Körperflüssigkeit HPV16L1 spezifische Antikörper nicht vorhanden sind oder unterhalb der Nachweisgrenze des Analysesystems (4) liegen.

6. Vorrichtung nach Anspruch 5, **dadurch gekennzeichnet, dass** das Behältnis (2) verschließbar ist.

7. Vorrichtung nach Anspruch 5 oder 6, **dadurch gekennzeichnet, dass** das Behältnis (2) eine Entnahme für die Patientenprobe, insbesondere kapillare Entnahme (10), aufweist.

8. Vorrichtung nach einem der Ansprüche 5 bis 7, **dadurch gekennzeichnet, dass** der Stoff aus kolloidalem Gold und/oder kolloidalem Silber, insbesondere in aggregierter Form, besteht.

9. Vorrichtung nach einem der Ansprüche 5 bis 8, **dadurch gekennzeichnet, dass** das Analysesystem (4) eine vorgegebenen Menge wenigstens eines Moleküls aufweist, welche sich an wenigstens einen Bestandteil des Konjugats bindet und welches an einer vorgegebenen zweiten Stelle oder einem vorgegebenen zweiten Bereich (28) des Trägermediums (16) fixiert ist, wobei in Fließrichtung (20) des aufgegebenen Substrates gesehen, die zweite Stelle oder der zweite Bereich (28) vorzugsweise nach der ersten Stelle oder dem ersten Bereich (26) angeordnet ist.

10. Behältnis (2) für eine Vorrichtung zur Durchführung eines Schnelltests zum qualitativen und/oder quantitativen Bestimmen von in Körperflüssigkeit enthaltenen Antikörpern gegen humane Papillomviren vom Typ 16 (HPV16),
wobei in dem Behältnis (2) ein Reagenz (14) im Wesentlichen aus einer vorgegebenen Menge an physiologisch wirkender Flüssigkeit und einer vorgegebenen Menge wenigstens eines Antigens enthalten ist und zu dem Reagenz (14) Körperflüssigkeit aus einer Patientenprobe in das Behältnis (2) einfüllbar ist,
wobei das Antigen aggregiert ist und bezüglich in der Körperflüssigkeit enthaltener Antikörper reaktiv ist, welche gegen das L1-Protein der HPV16 spezifisch sind,
wobei die Flüssigkeit auf das Antigen physiologisch wirkend ist, um die Reaktivität des Antigens in Bezug auf diese Antikörper aufrecht zu erhalten, und
wobei das Behältnis (2) ein Innengefäß (8) und ein das Innengefäß (8) umgebendes Gehäuse (6) aufweist und sich in dem Innengefäß (8) das Reagenz (14) befindet.

11. Analysesystem (4) für eine Vorrichtung zur Durchführung eines Schnelltests zum qualitativen und/oder quantitativen Bestimmen von in Körperflüssigkeit enthaltenen Antikörpern gegen humane Papillomviren vom Typ 16 (HPV16),
wobei das Analysesystem eingerichtet ist, eine vorgegebene Menge eines in einem Behältnis (2) befindlichen Substrates aufzunehmen und eine durch das Substrat bewirkte messbare und/oder von einem Benutzer wahrnehmbare Veränderung zu nutzen,
wobei das Substrat aus einem Reagenz (14) und Körperflüssigkeit aus einer Patientenprobe besteht und das Reagenz (14) im Wesentlichen aus einer vorgegebenen Menge an physiologisch wirkender Flüssigkeit und einer vorgegebenen Menge wenigstens eines Antigens besteht,
wobei das Antigen aggregiert ist und bezüglich in der Körperflüssigkeit enthaltener Antikörper reaktiv ist, welche gegen das L1-Protein der HPV16 spezifisch sind (HPV16L1 spezifische Antikörper), und die Flüssigkeit auf das Antigen physiologisch wirkend ist, um die Reaktivität des Antigens in Bezug auf diese Antikörper aufrecht zu erhalten,
wobei ferner das Analysesystem (4) umfasst:
• ein Konjugat, welches im Wesentlichen aus einer vorgegebenen Menge wenigstens eines Moleküls und einem Stoff besteht, der eine messbare und/oder von einem Benutzer wahrnehmbare Reaktion bewirkt oder bei räumlicher Häufung eine messbare und/oder von einem Benutzer wahrnehmbare Veränderung ergibt, wobei das wenigstens eine Molekül gegen das L1-Protein der HPV16 spezifisch ist und sich an das Antigen bindet, wenn das Antigen noch reaktiv ist,
• ein Trägermedium (16), an oder in dem die aufgegebene Menge des Substrates aus dem Behältnis (2) in Richtung zumindest einer Raumachse (20) fließbar ist,
• eine vorgegebene Menge wenigstens eines Fangmoleküls, welches an einer vorgegebenen ersten Stelle oder einem vorgegebenen ersten Bereich (26) des Trägermediums (16) fixiert ist und sich an das wenigstens eine Antigen bindet, wenn das Antigen noch reaktiv ist,
wobei das Fangmolekül und das Molekül des Konjugats derart zueinander identisch sind, dass beide eine Bindung mit dem Antigen eingehen, und das Antigen noch reaktiv ist, wenn in der Probe von Körperflüssigkeit HPV16L1 spezifische Antikörper nicht vorhanden sind oder unterhalb der Nachweisgrenze des Analysesystems (4) liegen.

## Claims

1. Rapid assay for qualitatively and/or quantitatively determining antibodies against human papillomaviruses of type 16 (HPV16) that are present in body fluid,
in which a sample of body fluid is mixed with a reagent (14) which substantially consists of a predefined amount of physiologically-acting liquid and a predefined amount of at least one antigen, the antigen being aggregated and being reactive with regard to antibodies which are present in the body fluid and which are specific against the L1 protein of HPV16 (HPV16L1-specific antibodies) and the liquid acting physiologically on the antigen in order to maintain the reactivity of the antigen with regard to said antibodies, and
in which the mixture is subsequently subjected to an analysis, the analysis using:
• a conjugate which substantially consists of a predefined amount of at least one molecule and of a substance which brings about a measurable and/or user-perceptible reaction or, in the case of spatial accumulation, yields a measurable and/or user-perceptible change, the at least one molecule being specific against the L1 protein of HPV16 and binding to the antigen when the antigen is still reactive,
• a support medium (16) on which or in which the delivered amount of the mixture is flowable in the direction of at least one spatial axis (20),
• a predefined amount of at least one capture molecule which is fixed at a predefined first site or a predefined first region (26) of the support medium (16) and binds to the at least one antigen when the antigen is still reactive,
the capture molecule and the molecule of the conjugate being identical to one another such that both form a bond with the antigen, and the antigen still being reactive when HPV16L1-specific antibodies are not present in the sample of body fluid or are below the detection limit of the method.

2. Rapid assay according to Claim 1, **characterized in that** a predefined amount of the mixture is subjected to the analysis.

3. Rapid assay according to Claim 1 or 2, **characterized in that** the amount of the at least one antigen is determined by a predefined detection limit for the amount to be determined of the HPV16L1-specific antibodies present in the body fluid sample.

4. Rapid assay according to any of the preceding claims, **characterized in that**, in the event of absent HPV16L1-specific antibodies in the sample or in the event of an amount of HPV16L1-specific antibodies in the sample that are below or in the region of a predefined detection limit for the HPV16L1-specific antibodies, a medium is changed in a user-perceptible manner and/or in a measurable manner, especially over a predefined planar section or a predefined volume.

5. Device for carrying out a rapid assay for qualitatively or quantitatively determining antibodies against human papillomaviruses of type 16 (HPV16) that are present in body fluid, especially according to any of the preceding claims,
comprising a container (2) which contains a reagent (14) substantially composed of a predefined amount of physiologically-acting liquid and a predefined amount of at least one antigen and into which it is possible to fill body fluid from a patient sample towards the reagent (14), the antigen being aggregated and being reactive with regard to antibodies which are present in the body fluid and which are specific against the L1 protein of HPV16 (HPV16L1-specific antibodies), and the liquid acting physiologically on the antigen in order to maintain the reactivity of the antigen with regard to said antibodies, and
comprising an analysis system (4) which is designed to accommodate a predefined amount of a substrate situated in the container (2) that is formed from the reagent (14) and the patient sample and which uses a measurable and/or user-perceptible change brought about by the substrate, the analysis system (4) comprising:
• a conjugate which substantially consists of a predefined amount of at least one molecule and of a substance which brings about a measurable and/or user-perceptible reaction or, in the case of spatial accumulation, yields a measurable and/or user-perceptible change, the at least one molecule being specific against the L1 protein of HPV16 and binding to the antigen when the antigen is still reactive,
• a support medium (16) on which or in which the delivered amount of the substrate from the container (2) is flowable in the direction of at least one spatial axis (20),
• a predefined amount of at least one capture molecule which is fixed at a predefined first site or a predefined first region (26) of the support medium (16) and binds to the at least one antigen when the antigen is still reactive,
the capture molecule and the molecule of the conjugate being identical to one another such that both form a bond with the antigen, and the antigen still being reactive when HPV16L1-specific antibodies are not present in the sample of body fluid or are below the detection limit of the analysis system (4).

6. Device according to Claim 5, **characterized in that** the container (2) is closable.

7. Device according to Claim 5 or 6, **characterized in that** the container (2) has a removal point for the patient sample, especially a capillary removal point (10).

8. Device according to any of Claims 5 to 7, **characterized in that** the substance consists of colloidal gold and/or colloidal silver, especially in aggregated form.

9. Device according to any of Claims 5 to 8, **characterized in that** the analysis system (4) comprises a predefined amount of at least one molecule which binds to at least one constituent of the conjugate and which is fixed at a predefined second site or a predefined second region (28) of the support medium (16), the second site or the second region (28) preferably being arranged after the first site or the first region (26) when viewed in the flow direction (20) of the delivered substrate.

10. Container (2) for a device for carrying out a rapid assay for qualitatively and/or quantitatively determining antibodies against human papillomaviruses of type 16 (HPV16) that are present in body fluid,
the container (2) containing a reagent (14) substantially composed of a predefined amount of physiologically-acting liquid and a predefined amount of at least one antigen and it being possible to fill body fluid from a patient sample into the container (2) towards the reagent (14),
the antigen being aggregated and being reactive with regard to antibodies which are present in the body fluid and which are specific against the L1 protein of HPV16,
the liquid acting physiologically on the antigen in order to maintain the reactivity of the antigen with regard to said antibodies, and
the container (2) comprising an inner vessel (8) and a housing (6) surrounding the inner vessel (8) and the reagent (14) being situated in the inner vessel (8).

11. Analysis system (4) for a device for carrying out a rapid assay for qualitatively and/or quantitatively determining antibodies against human papillomaviruses of type 16 (HPV16) that are present in body fluid,
the analysis system being set up to accommodate a predefined amount of a substrate situated in a container (2) and to use a measurable and/or user-perceptible change brought about by the substrate,
the substrate consisting of a reagent (14) and body fluid from a patient sample and the reagent (14) substantially consisting of a predefined amount of physiologically-acting liquid and a predefined amount of at least one antigen,
the antigen being aggregated and being reactive with regard to antibodies which are present in the body fluid and which are specific against the L1 protein of HPV16 (HPV16L1-specific antibodies), and the liquid acting physiologically on the antigen in order to maintain the reactivity of the antigen with regard to said antibodies,
the analysis system (4) further comprising:
• a conjugate which substantially consists of a predefined amount of at least one molecule and of a substance which brings about a measurable and/or user-perceptible reaction or, in the case of spatial accumulation, yields a measurable and/or user-perceptible change, the at least one molecule being specific against the L1 protein of HPV16 and binding to the antigen when the antigen is still reactive,
• a support medium (16) on which or in which the delivered amount of the substrate from the container (2) is flowable in the direction of at least one spatial axis (20),
• a predefined amount of at least one capture molecule which is fixed at a predefined first site or a predefined first region (26) of the support medium (16) and binds to the at least one antigen when the antigen is still reactive,
the capture molecule and the molecule of the conjugate being identical to one another such that both form a bond with the antigen, and the antigen still being reactive when HPV16L1-specific antibodies are not present in the sample of body fluid or are below the detection limit of the analysis system (4).

## Revendications

1. Test rapide pour la détermination qualitative et/ou quantitative d'anticorps contre des virus du papillome humain du type 16 (VPH16) contenus dans un liquide corporel,
dans lequel un échantillon de liquide corporel est mélangé avec un réactif (14), qui est essentiellement constitué par une quantité prédéfinie de liquide à effet physiologique et par une quantité prédéfinie d'au moins un antigène, l'antigène étant aggloméré et réactif par rapport aux anticorps, contenus dans le liquide corporel, qui sont spécifiques à la protéine L1 du VPH16 (anticorps spécifiques à VPH16L1) et le liquide ayant un effet physiologique sur l'antigène pour maintenir la réactivité de l'antigène par rapport à ces anticorps et
dans lequel le mélange est ensuite introduit dans une analyse, l'analyse utilisant :
• un conjugué qui est essentiellement constitué par une quantité prédéfinie d'au moins une molécule et par une substance qui provoque une réaction mesurable et/ou observable par un utilisateur ou qui provoque, lors d'une concentration spatiale, une modification mesurable et/ou observable par un utilisateur, ladite au moins une molécule étant spécifique à la protéine L1 du VPH16 et se liant à l'antigène lorsque l'antigène est encore réactif,
• un milieu support (16) sur lequel ou dans lequel la quantité appliquée de mélange peut s'écouler dans le sens d'au moins un axe spatial (20),
• une quantité prédéfinie d'au moins une molécule piège, qui est fixée en un premier site prédéfini ou en une première zone (26) prédéfinie du milieu support (16) et qui se lie audit au moins un antigène, lorsque l'antigène est encore réactif,
la molécule piège et la molécule de conjugué présentant une identité l'une par rapport à l'autre telle que les deux participent à une liaison avec l'antigène et l'antigène étant encore réactif lorsque, dans l'échantillon de liquide corporel, des anticorps spécifiques à la VPH16L1 ne sont pas présents ou sont inférieurs à la limite de détection du procédé.

2. Test rapide selon la revendication 1, **caractérisé en ce qu'**une quantité prédéfinie du mélange est introduite dans l'analyse.

3. Test rapide selon la revendication 1 ou 2, **caractérisé en ce que** la quantité dudit au moins un antigène est déterminée par une limite de détection prédéfinie pour la quantité à déterminer des anticorps spécifiques à la VPH16L1 contenus dans l'échantillon de liquide.

4. Test rapide selon l'une quelconque des revendications précédentes, **caractérisé en ce que**, lorsqu'il n'y a pas d'anticorps spécifiques à la VPH16L1 ou dans le cas d'une quantité d'anticorps spécifiques à la VPH16L1 dans l'échantillon qui est inférieure à ou située dans la plage d'une limite de détection prédéfinie pour les anticorps spécifiques à la VPH16L1, un milieu, en particulier sur une section surfacique prédéfinie ou sur un volume prédéfini, est modifié de manière observable par un utilisateur et/ou mesurable.

5. Dispositif pour la réalisation d'un test rapide pour la détermination qualitative ou quantitative d'anticorps contre des virus du papillome humain du type 16 (HPV16) contenus dans un liquide corporel, en particulier selon l'une quelconque des revendications précédentes, présentant un récipient (2) dans lequel est contenu un réactif (14), essentiellement constitué par une quantité prédéfinie de liquide à effet physiologique et par une quantité prédéfinie d'au moins un antigène, et dans lequel du liquide corporel provenant d'un échantillon de patient peut être ajouté au réactif (14), l'antigène étant aggloméré et réactif par rapport aux anticorps, contenus dans le liquide corporel, qui sont spécifiques à la protéine L1 du VPH16 (anticorps spécifiques à VPH16L1) et le liquide ayant un effet physiologique sur l'antigène pour maintenir la réactivité de l'antigène par rapport à ces anticorps et
présentant un système d'analyse (4), qui est conçu pour recevoir une quantité prédéfinie d'un substrat, constitué par le réactif (14) et l'échantillon de patient, se trouvant dans le récipient (2) et qui utilise une modification mesurable et/ou observable par un utilisateur provoqué par le substrat, le système d'analyse (4) présentant :
• un conjugué qui est essentiellement constitué par une quantité prédéfinie d'au moins une molécule et par une substance qui provoque une réaction mesurable et/ou observable par un utilisateur ou qui provoque, lors d'une concentration spatiale, une modification mesurable et/ou observable par un utilisateur, ladite au moins une molécule étant spécifique à la protéine L1 du VPH16 et se liant à l'antigène lorsque l'antigène est encore réactif,
• un milieu support (16) sur lequel ou dans lequel la quantité appliquée de substrat provenant du récipient (2) peut s'écouler dans le sens d'au moins un axe spatial (20),
• une quantité prédéfinie d'au moins une molécule piège, qui est fixée en un premier site prédéfini ou en une première zone (26) prédéfinie du milieu support (16) et qui se lie audit au moins un antigène, lorsque l'antigène est encore réactif,
la molécule piège et la molécule de conjugué présentant une identité l'une par rapport à l'autre telle que les deux participent à une liaison avec l'antigène et l'antigène étant encore réactif lorsque, dans l'échantillon de liquide corporel, des anticorps spécifiques à la VPH16L1 ne sont pas présents ou sont inférieurs à la limite de détection du système d'analyse (4).

6. Dispositif selon la revendication 5, **caractérisé en ce que** le récipient (2) peut être fermé.

7. Dispositif selon la revendication 5 ou 6, **caractérisé en ce que** le récipient (2) présente un site de prélèvement pour l'échantillon de patient, en particulier un site de prélèvement capillaire (10).

8. Dispositif selon l'une quelconque des revendications 5 à 7, **caractérisé en ce que** la substance est constituée par de l'or colloïdal et/ou de l'argent colloïdal, en particulier sous forme agglomérée.

9. Dispositif selon l'une quelconque des revendications 5 à 8, **caractérisé en ce que** le système d'analyse (4) présente une quantité prédéfinie d'au moins une molécule, qui se lie à au moins un constituant du conjugué et qui est fixée en un deuxième site prédéfini ou dans une deuxième zone prédéfinie (28) du milieu support (16), le deuxième site ou la deuxième zone (28), vu(e) dans le sens d'écoulement (20) du substrat appliqué, étant de préférence disposé(e) en aval du premier site ou de la première zone (26).

10. Récipient (2) pour un dispositif pour la réalisation d'un test rapide pour la détermination qualitative et/ou quantitative d'anticorps contre des virus du papillome humain du type 16 (HPV16) contenus dans un liquide corporel,
un réactif (14), essentiellement constitué par une quantité prédéfinie de liquide à effet physiologique et par une quantité prédéfinie d'au moins un antigène, étant contenu dans le récipient (2) et un liquide corporel provenant d'un échantillon de patient pouvant être ajouté au réactif (14) dans le récipient (2),
l'antigène étant aggloméré et réactif par rapport aux anticorps, contenus dans le liquide corporel, qui sont spécifiques à la protéine L1 du VPH16 et
le liquide ayant un effet physiologique sur l'antigène pour maintenir la réactivité de l'antigène par rapport à ces anticorps et
le récipient (2) présentant un récipient interne (8) et un boîtier (6) entourant le récipient interne (8) et le réactif (14) se trouvant dans le récipient interne (8).

11. Système d'analyse (4) pour un dispositif pour la réalisation d'un test rapide pour la détermination qualitative et/ou quantitative d'anticorps contre des virus du papillome humain du type 16 (HPV16) contenus dans un liquide corporel,
le système d'analyse étant conçu pour recevoir une quantité prédéfinie d'un substrat se trouvant dans un récipient (2) et pour utiliser une modification mesurable et/ou observable par un utilisateur provoquée par le substrat,
le substrat étant constitué par un réactif (14) et du liquide corporel provenant d'un échantillon de patient et le réactif (14) étant essentiellement constitué par une quantité prédéfinie de liquide à effet physiologique et par une quantité prédéfinie d'au moins un antigène, l'antigène étant aggloméré et réactif par rapport aux anticorps, contenus dans le liquide corporel, qui sont spécifiques à la protéine L1 du VPH16 (anticorps spécifiques à VPH16L1) et le liquide ayant un effet physiologique sur l'antigène pour maintenir la réactivité de l'antigène par rapport à ces anticorps,
le système d'analyse (4) comprenant en outre:
• un conjugué qui est essentiellement constitué par une quantité prédéfinie d'au moins une molécule et par une substance qui provoque une réaction mesurable et/ou observable par un utilisateur ou qui provoque, lors d'une concentration spatiale, une modification mesurable et/ou observable par un utilisateur, ladite au moins une molécule étant spécifique à la protéine L1 du VPH16 et se liant à l'antigène lorsque l'antigène est encore réactif,
• un milieu support (16) sur lequel ou dans lequel la quantité appliquée de substrat provenant du récipient (2) peut s'écouler dans le sens d'au moins un axe spatial (20),
• une quantité prédéfinie d'au moins une molécule piège, qui est fixée en un premier site prédéfini ou en une première zone (26) prédéfinie du milieu support (16) et qui se lie audit au moins un antigène, lorsque l'antigène est encore réactif,
la molécule piège et la molécule de conjugué présentant une identité l'une par rapport à l'autre telle que les deux participent à une liaison avec l'antigène et l'antigène étant encore réactif lorsque, dans l'échantillon de liquide corporel, des anticorps spécifiques à la VPH16L1 ne sont pas présents ou sont inférieurs à la limite de détection du système d'analyse (4) .
